# EUROPÄISCHE PATENTANMELDUNG

(11) **EP 0 995 443 A1**
(43) Veröffentlichungstag der Anmeldung: **26.04.2000**
(21) Anmeldenummer: 99120377.9
(22) Anmeldetag: 13.10.1999
(51) Int. Cl.: A61K 35/78, A61P 17/02, A61P 31/22

(54) **Präparat zur Behandlung von Brandwunden, Hautabschürfungen und Herpes**

(30) Priorität: 20.10.1998 DE 19848336
(71) Anmelder: Bacciu, Maria, Sardinien (IT)
(72) Erfinder: Bacciu, Maria, Sardinien (IT)
(74) Vertreter: Freischem, Werner, Dipl.-Ing.

(57) **Zusammenfassung**

Präparat zur Behandlung von Brandwunden, Hautabschürfungen und Herpes (z.B. Herpes zoster oder Herpes simplex), bestehend aus dem vergorenen Saft aus der Wurzel der Pflanze Asphodelus albus. Dieser vergorene Saft dient zur Behandlung von Brand- oder Schürfwunden und zum Abheilen von durch Herpes-Infektionen (Herpes simplex oder Herpes zoster) entstandenen Entzündungen und Hautschäden.

## Beschreibung

Die Erfindung bezieht sich auf ein Präparat zur Behandlung von Brand- oder Schürfwunden sowie von Hautverletzungen, die durch Entzündungen insbesondere durch Herpes-Viren verursacht sind.

Die Erfinderin hat erkannt, daß Hautverletzungen wie Brand- und Schürfwunden schnell und insbesondere narbenfrei abheilen, wenn diese mittels eines vergorenen Saftes aus der Wurzel der Pflanze Asphodelus albus behandelt werden.

Bei der Pflanze Asphodelus albus - auf deutsch Affodill - handelt es sich um ein in Südeuropa, insbesondere in Spanien, Sardinien, Sizilien und Albanien vorkommendes Liliengewächs mit hochstengeligen Ähren oder Rispen weißer oder gelber Blüten. Es ist bekannt, daß die Wurzel dieser Pflanze Inulin enthält, ein Stoff, der bei Menstruationsbeschwerden und als Diuretikum Anwendung findet.

Zur Herstellung des Präparates nach der Erfindung wird die Affodill-Pflanze bis zu einer Tiefe von ca. 30 cm freigegraben und aus dem Erdreich entnommen. Der oberirdische Teil der Pflanze wird abgeschnitten und beseitigt. Anschließend wird die spindelförmige Wurzel, die im oberflächennahen Ansatz einen Durchmesser von bis zu 4 cm haben kann, mit Wasser gesäubert, bis sie vollständig von Erdbestandteilen gereinigt ist. Danach wird die dünne Außenhaut abgelöst und nochmals mit Wasser nachgespült. Die geschälte Wurzel wird anschließend in kleine Stückchen geschnitten und zum Pürieren in eine hierzu geeignete Maschine gefüllt. Die pürierte Masse wird dann in ein Tuch, z.B. ein sauberes Baumwolltuch, gegeben und ausgepreßt. Die austretende klare Flüssigkeit, die eine gelbliche Färbung aufweist, wird in zuvor sterilisierte Behälter, z.B. getönte Glasflaschen, gefüllt. Die Behälter werden nicht verschlossen, so daß in einem Raum mit geringer Luftfeuchtigkeit und bei Zimmertemperatur eine aerobe Gärung in Gang gesetzt wird. Nach etwa 6 - 7 Tagen hat sich an der Oberfläche der Flüssigkeit das Ferment (Gärungspilz) abgesetzt. Nun kann das Präparat zur Behandlung von Wunden und dergleichen eingesetzt werden.

Wie schon während der Gärung bleiben die Gefäße in einem Raum mit normaler Zimmertemperatur und geringer Luftfeuchtigkeit. Weiterhin darf das Präparat keiner direkten Sonneneinstrahlung ausgesetzt werden.

Das sich auf dem Flüssigkeitsspiegel absetzende Ferment kann bis zur ersten Behandlung in den Flaschen verbleiben oder aber nach Ablauf von 6-7 Tage entfernt werden. Die Glasgefäße werden zur Aufbewahrung mit einem Pfropfen aus Naturkork verschlossen. Zuvor wird jedoch eine Kerbe in den Pfropfen geschnitten, so daß im Flascheninneren kein Überdruck entstehen kann.

Die Haltbarkeit des Präparates, dem keine Konservierungsmittel zugesetzt wurden, beträgt 3 - 4 Monate. Wenn das Präparat einen säuerlichen Geruch annimmt, sollte es nicht weiter verwendet werden.

Je früher mit der Behandlung eingesetzt wird desto größer sind die Erfolgsaussichten für eine gute, d.h. vollständige Heilung ohne Verbleib von Narben. Der günstigste Zeitpunkt ist direkt nach erfolgter Verletzung durch Verbrennung oder Sturz. Das gleiche gilt für jede Art von Herpesausbildungen, deren frühzeitige Erkennung und Behandlung den besten Erfolg verspricht. Dennoch läßt auch ein späterer Behandlungsbeginn, wie zum Beispiel bei Herpes im fortgeschrittenen Stadium oder bei Verbrennungen und Hautabschürfungen erst Stunden oder Tage nach erfolgter Verletzung, noch gute Heilungserfolge erwarten.

Bei Verbrennungen und Hautschürfungen muß die Wunde vor Beginn der Medikamentengabe gründlich gereinigt werden und - soweit möglich - alle Brandrückstände und verbrannte Haut beseitigt werden. Die Reinigung erfolgt mit einem Desinfektionsmittel, welches vorsichtig über die Wunde gestrichen wird.

Je nach Zustand der Wunde und dem Fortschritt des Heilungsprozesses sind weitere Desinfektionen im Laufe der Behandlung erforderlich. Vor jeder Medikamentengabe ist zu entscheiden, ob erneut desinfiziert werden muß.

Nach erfolgter Desinfektion sollte die Wunde kurz trocknen. Zur Beschleunigung dieses Prozesses kann hierzu ein Ventilator verwendet werden.

Vor Aufbringen des Präparates ist darauf zu achten, daß die Wunde möglichst in eine waagerechte Lage kommt. Dann wird das Mittel mit Hilfe einer Pipette direkt auf die Wunde gegeben und mit einer Feder verteilt. Dabei wird die gesamte Wunde möglichst gleichmäßig benetzt. Im Anschluß an einen Auftrag muß die Wunde kurz trocknen. Danach kann das Präparat noch ein- bis dreimal aufgetragen werden.

Nach erfolgter Verabreichung des Präparates ist die Wunde offen zu belassen.

Je nach dem Grad der Verbrennung oder der Hautschürfung bzw. nach der Art und dem Stadium von Herpes und nach dem Fortschritt des Heilungsprozesses kann das Präparat 1 - 2 x täglich verabreicht werden. Dabei können 1 bis 4 Schichten des Präparates aufgetragen werden. Je nach Schwere der Verletzung kann die Behandlung 10 - 20 Tage dauern.

Da die Wunde während der gesamten Behandlungsdauer ohne Schutz ist, muß dafür gesorgt werden, daß die Wunde - soweit es die Umstände zulassen - unberührt bleibt. Ein Kontakt mit Wasser ist zu vermeiden. Auch soll die Wunde vor direktem Sonnenlicht geschützt sein.

## Patentansprüche

1. Präparat zur Behandlung von Brandwunden, Hautabschürfungen und Herpes (z.B. Herpes zoster oder Herpes simplex), **gekennzeichnet** durch den vergorenen Saft aus der Wurzel der Pflanze Asphodelus albus.

2. Verwendung des vergorenen Saftes aus der Wurzel der Pflanze Asphodelus albus zur Behandlung von Brand- oder Schürfwunden und zum Abheilen von durch Herpes-Infektionen (Herpes simplex oder Herpes zoster) entstandenen Entzündungen und Hautschäden.

3. Verfahren zur Herstellung des Präparates nach Anspruch 1, **dadurch gekennzeichnet**, daß nach Freigraben der Pflanze Asphodelus albus der oberirdische Teil der Pflanze abgeschnitten und der Wurzelteil gereinigt, geschält, in kleine Stücke geschnitten und püriert wird und die so entstandene Masse z.B. in einem Tuch ausgepreßt wird und der abgepreßte Saft einer aeroben Gärung unterzogen wird, bis nach einigen Tagen das Ferment sich auf der Oberfläche der Flüssigkeit absetzt und entfernt wird.
